# EUROPEAN PATENT APPLICATION

(11) **EP 4 667 000 A1**
(43) Date of publication of application: **24.12.2025**
(21) Application number: 24182709.6
(22) Date of filing: 17.06.2024
(51) Int. Cl.: A61K 9/08, A61K 47/36, A61K 47/10

(54) **HYPEROSMOLAR OPHTHALMIC COMPOSITION COMPRISING CARBOXYMETHYL CHITOSAN**

(71) Applicant: Omnivision GmbH, 82178 Puchheim (DE)
(72) Inventor: MÜLLER, Saskia, 82178 Puchheim (DE); MELZER, Benedikt, 82178 Puchheim (DE); HOFFMANN, Patrick, 82178 Puchheim (DE); HOFFMANN, Burkhardt, 82178 Puchheim (DE); KOHL, Tobias, 82178 Puchheim (DE)
(74) Representative: Wächtershäuser & Hartz Patentanwaltspartnerschaft mbB

(57) **Abstract**

The invention provides a hyperosmolar ophthalmic composition comprising carboxymethyl chitosan. The ophthalmic composition can be used for treating corneal edema and for suppressing bacterial growth on the eye.

## Description

### FIELD OF THE INVENTION

The invention relates to an ophthalmic composition, notably an aqueous hyperosmolar ophthalmic composition, suitable for the treatment of, inter alia, corneal edema and for preventing or decreasing the risk for bacterial infection and/or bacterial growth on the cornea. The invention also provides an ophthalmic composition, notably an aqueous hyperosmolar ophthalmic composition, for use in a method of treating or reducing corneal swelling or fluid accumulation, including stromal corneal edema or epithelial corneal edema, especially after surgical intervention, such as cataract surgery or corneal transplantation; or for restoring osmotic balance of the cornea, especially after surgical intervention, such as cataract surgery or corneal transplantation, The invention also provides a method of treating or reducing corneal edema, corneal swelling or fluid accumulation, including stromal corneal edema or epithelial corneal edema, especially after surgical intervention, such as cataract surgery or corneal transplantation; or for restoring osmotic balance of the cornea, especially after surgical intervention such as cataract surgery or corneal transplantation. The invention further relates to an ophthalmic composition for use in a method of decreasing the risk for bacterial infection and/or bacterial growth on the cornea, notably after corneal surgery.

### BACKGROUND OF THE INVENTION

Corneal edema is the swelling of the cornea following ocular surgery, trauma, infection, inflammation as well as a secondary result of various ocular diseases. Corneal edema can also occur following over-wear of certain types of contact lenses. Corneal edema leads to a thickening of the cornea. The cornea is part of the eye's focusing system that transmits and focuses light into the eye. When the cornea thickens, it may impair transmission of light, which may decrease vision. The thickening of the cornea can also lead to a loss of corneal transparency and a lowering of the visual acuity.

Corneal edema may be treated by topical application of hypertonic eye drops, generally containing hyperosmolar concentrations of sodium chloride. US 2017/0128484 A1 (Horus Pharma) describes an aqueous hyperosmolar solution containing hydroxypropylmethylcellulose (HPMC) and hyaluronic acid. The exemplified solution contains 5 % by weight sodium chloride, 0.25 % by weight HPMC, 0.15 % by weight sodium hyaluronate and citrate buffer. The solution is said to have a pH between 6.2 and 6.8 and an osmolality of about 1750 mOsm/kg. A commercial product of this type is ODM5^{™} of Horus Pharma.

The osmolarity of tears is generally within the range of about 280 to 330 mOsm/l. Thus, eye drops having an osmolarity or osmolality of a solution containing about 5 % by weight sodium chloride is non-physiological to the eye. Moreover, upon application to the eye, cells of the corneal layers, notably cells of the outer epithelial layer, suffer an osmotic shock, which can potentially be damaging to corneal tissue. Therefore, hyperosmolar eye dops generally contain components such as hydrophilic polymers that advantageously have a protecting and/or healing effect on the cornea. An example of such hydrophilic polymer is hyaluronic acid or salts thereof that is also contained in ODM5^{™}. However, hyperosmolar eye dops generally have no or little antimicrobial activity.

Ocular surgery, such as lens replacement, bears a risk of bacterial infection of the eye undergoing surgery. In contrast to bacterial eye infections on the outside or on the surface of the eye, such as conjunctivitis, keratitis and blepharitis, ocular surgery bears a risk of bacteria entering the eye ball, which may lead to endophthalmitis. Endophthalmitis is very difficult to treat and frequently leads to loss of the affected eye. Therefore, avoiding bacterial growth and/or infection is particularly important during and after ocular surgery.

Departing from the prior art, it is an object of the invention to provide an agent and/or method for reducing the risk of bacterial infection and bacterial growth on the cornea after ocular surgery and other trauma to the eye. It is a further object of the invention to provide an agent and/or method for treating corneal edema, in particular after ocular surgery, that at the same time reduces the risk of bacterial infection and/or bacterial growth on the cornea and adjacent tissues. It is a further object of the invention to provide an agent and/or method for treating corneal edema and reducing the risk of bacterial infection (e.g. also including endophthalmitis).

### SUMMARY OF THE INVENTION

In order to accomplish these objects, the invention provides:
1) A hyperosmolar ophthalmic composition comprising carboxymethyl chitosan.
2) The hyperosmolar ophthalmic composition according to 1), comprising from 0.05 to 3 % by weight, preferably from 0.1 to 1 % by weight, of said carboxymethyl chitosan.
3) The ophthalmic composition according to 1) or 2), wherein said carboxymethyl chitosan has a degree of deacetylation of at least 50 %, preferably from 60 to 98 % by weight.
4) The ophthalmic composition according to any one of 1) to 3), wherein said carboxymethyl chitosan has an average degree of carboxymethylation of from 0.5 to 2.5, preferably 0.7 to 2.0, more preferably 1.0 to 1.8 mol per monomer unit of said carboxymethyl chitosan.
5) The ophthalmic composition according to any one of 1) to 4), wherein said carboxymethyl chitosan has a weight average molecular weight of from 100,000 to 700,000, preferably from 150,000 to 500,000 Da.
6) The ophthalmic composition according to any one of 1) to 5), further comprising a buffer selected from citric acid or salts thereof, tris, histidine, imidazole, and phosphoric acid or salts thereof, preferably tris.
7) The ophthalmic composition according to any one of 1) to 6), further comprising a panthenol, such as dexpanthenol.
8) The ophthalmic composition according to any one of 1) to 7), further comprising at least one viscosity regulating agent that may be selected from the group consisting of a hydroxyalkylated cellulose, chondroitin sulfate, polyacrylic acid, polyvinyl alcohol, polyethylene glycol, polysaccharides, polyvinylpyrrolidone, heparin, hyaluronic acid, cross-linked hyaluronic acid, and salts thereof, preferably wherein the one or more viscosity regulating agent is or comprises hyaluronic acid or a salt thereof.
9) The ophthalmic composition according to any one of 1) to 8), comprising at least one osmolarity agent.
10) The ophthalmic composition according to 9), wherein the at least one osmolarity agent is selected from the group consisting of sodium chloride, potassium chloride, trehalose, and sugar alcohols.
11) The ophthalmic composition according to 10), wherein the sugar alcohols are selected from the group consisting of glycerol, mannitol, and sorbitol.
12) The ophthalmic composition according to 9), wherein the at least one osmolarity agent is or comprises sodium chloride and optionally one or more sugar alcohol.
13) The ophthalmic composition according to any one of 1) to 12), comprising, as osmolarity agent, sodium chloride in a concentration of from 2.0 to 6.5 % by weight, preferably from 3.0 to 6.0 % by weight, more preferably 4.0 to 5.5 % by weight, even more preferably from 4.5 to 5.3 % by weight, and most preferably about 5 % by weight.
14) The ophthalmic composition according to any one of 1) to 13), comprising, as osmolarity agent, glycerol in an amount of from 1.0 to 3.5 %, preferably from 2.0 to 3.0 %, and even more preferably from 2.3 to 2.7 % by weight.
15) The ophthalmic composition according to any one of 1) to 14), wherein the ophthalmic composition is an aqueous solution having an osmolarity of at least 1000 mOsm/L, preferably at least 1400 mOsm/L, or having an osmolality of at least 1000 mOsm/kg, preferably at least 1400 mOsm/kg.
16) The ophthalmic composition according to any one of 1) to 15), comprising:
   (i) said carboxymethyl chitosan,
   (ii) sodium chloride
   (iii) dexpanthenol,
   (iv) a buffer,
   (v) water,
   wherein the osmolarity of the ophthalmic composition is at least 1000 mOsm/L or the osmolality of the ophthalmic composition is at least 1000 mOsm/kg.
17) The ophthalmic composition according to 16), comprising:
   (i) from 0.05 to 3 % by weight, preferably from 0.1 to 1 % by weight, of said carboxymethyl chitosan,
   (ii) from 3.0 to 6.5 % by weight, preferably from 3.5 to 6.0 % by weight sodium chloride,
   (iii) optionally from 0.2 to 3.0 % w/v dexpanthenol, preferably 0.3 to 2.5 % w/v dexpanthenol,
   (iv) 5 to 100 mM, preferably 10 to 50 mM, of said buffer.
18) The ophthalmic composition according to any one of 1) to 17), comprising:
   (i) said carboxymethyl chitosan,
   (ii) sodium chloride
   (iii) taurine
   (iv) a buffer,
   (v) water,
   wherein the calculated osmolarity of the ophthalmic composition is at least 1000 mOsm/L.
19) The ophthalmic composition according to 18), comprising:
   (i) from 0.05 to 3 % by weight, preferably from 0.1 to 1 % by weight, of said carboxymethyl chitosan,
   (ii) from 3.0 to 6.5 % by weight, preferably from 3.5 to 6.0 % by weight sodium chloride,
   (iii) 0.1 to 3.0 % w/v taurine, preferably 0.3 to 2.0 % w/v taurine,
   (iv) 5 to 100 mM, preferably 10 to 50 mM, of said buffer.
20) The ophthalmic composition according to any one of 1) to 19), containing dexpanthenol and taurine.
21) The ophthalmic composition according to any one of 1) to 20), having a pH of from 6.5 to 7.6; and/or a dynamic viscosity of, at a shear rate of 100 s⁻¹, from 2 to 1000 mPa s, preferably of from 3 to 500 mPa s, and more preferably from 4 to 200 mPa·s at 25°C, determined using a rotational viscometer.
22) The ophthalmic composition according to any one of 1) to 21), which contains no or less than 0.001 % by weight of a preservative, preferably contains no or less than 0.001 % by weight of quaternary ammonium compounds, such as benzalkonium chloride.
23) The ophthalmic composition according to any one of 1) to 22), further comprising heparin or a salt thereof, such as in an amount of from 500 to 2000 I.U. / mL, preferably from 1000 to 1600 I.U. / mL, more preferably of from 1100 to 1500 I.U. / mL, even more preferably of from 1200 to 1400 I.U / mL, and most preferably from 1250 to 1350 I.U. / mL of the ophthalmic composition.
24) The ophthalmic composition according any one of the preceding items for use in ophthalmology, preferably for use in a method of treating or reducing corneal swelling or fluid accumulation of any origin, including cataract surgery, corneal transplantation, stromal corneal edema or epithelial corneal edema, or for restoring osmotic balance of the cornea, especially after surgical intervention such as cataract surgery or corneal transplantation.
25) A method of treating or reducing corneal edema, including stromal corneal edema, epithelial corneal edema, corneal osmotic imbalance, post-surgical corneal osmotic imbalance, and corneal swelling or fluid accumulation of any origin (including after cataract surgery or corneal transplantation), the method comprising applying the ophthalmic composition according to any one of the preceding items to the surface of an eye of a patient.
26) The ophthalmic composition according any one of the preceding items for use in a method of treating corneal edema and of preventing or reducing the risk of bacterial infection and/or bacterial growth on the cornea.

The inventors have tried to solve the above-mentioned technical problems and have found that certain chitosan derivatives have an antimicrobial activity. The inventors have surprisingly found that carboxymethyl chitosan (CMCh) has a particular strong bacterial growth suppressing effect when present in high osmolar solutions, notably in solutions of high salt concentrations. It was particularly surprising to find that the bacterial growth suppressing effect was higher than the sum of the separate bacterial growth suppressing effects of high osmolar salt solutions and CMCh solutions. Thus, the inventors have found a synergistic bacterial growth suppressing effect of high osmolarity and CMCh. The bacteria on which these effects were found are bacteria that are relevant for ocular eye infections including for endophthalmitis. The inventors have thus recognized that high osmolar solutions containing CMCh can be used for treating corneal edema and at the same time reduce the risk of bacterial infection or bacterial growth on the treated area, notably on the cornea. The inventors have further recognised that high osmolar solutions containing CMCh can be used treating corneal edema and at the same time for preventing bacterial infection of the eye, such as endophthalmitis.

### BRIEF DESCRIPTION OF THE DRAWINGS

Fig. 1 Growth curves of *P*. *aeruginosa* in CMCh-containing test solutions as described in Example 1 and in comparative test solutions. The test solutions are indicated.
Fig. 2 Growth curves of S. *aureus* in CMCh-containing test solutions as described in Example 1 and in comparative test solutions.

### DETAILED DESCRIPTION OF THE INVENTION

The hyperosmolar ophthalmic composition of the present invention is generally an aqueous hyperosmolar ophthalmic composition. The present invention provides a hyperosmolar ophthalmic composition comprising carboxymethyl chitosan (in the following: CMCh).

The term "ophthalmic composition" refers to a composition suitable for use in ophthalmology, notably suitable for topical application to the surface of the eyes. The ophthalmic composition of the invention is generally aqueous because it contains water. Examples of the ophthalmic composition are solutions, emulsions, and dispersions. Generally, the hyperosmolar ophthalmic composition of the invention is a solution of its components in water. Examples of the ophthalmic compositions are eye drops, ophthalmic gels, and ophthalmic creams. Eye drops are most preferred.

Herein, the term "hyperosmolar" relates to aqueous compositions having an osmolarity or osmolality higher than that generally found in tears, i.e. higher than 300 mOsm/L or 300 mOsm/kg. The ophthalmic composition of the present invention generally has an osmolarity of at least 700 mOsm/L or an osmolality of at least 700 mOsm/kg.

Osmolarity is the concentration of solute(s) in a solution and is defined as the number of osmoles (Osm) of solute per liter of solution in unit Osm/L or mOsm/L. Osmolality is the concentration of solutes in a solution and is defined as the number of osmoles (Osm) of solute per kilogram of solvent in unit Osm/kg or mOsm/kg.

The osmole (Osm) is a unit of measurement which defines the number of moles of solute(s) that contribute(s) to the osmotic pressure of a solution. The osmolality (and osmolarity) of small molecules can generally be calculated from their concentration in solution. In the case of simple salts, such as sodium chloride, which dissociate essentially completely in water, both the (sodium) cation and the (chloride) anion contribute to the osmolality and osmolarity which can be calculated approximately from the sum of the concentrations of cations and anions. In the case of complex salts such as sodium phosphate, which partially dissociate in water, the osmolality and osmolarity depend on concentration and pH. Polymeric solutes contribute little to osmolality and osmolarity, but counter-ions of charged groups in polymers may contribute significantly to osmolality and osmolarity. Therefore, herein, osmolality is determined experimentally, and numerical values of osmolality are measured values according to Ph. Eur. 2.2.35.

Osmolality and osmolarity of a given aqueous composition are linked by the density of the composition and the concentration of the solutes: osmolarity = osmolality × (density of solution (kg/L) - mass concentration of solutes (kg/L)). Herein, the osmolarity of a solution is determined at a temperature of 25°C, unless otherwise specified.

The term "osmolarity agent" (may also be referred to as "hyperosmolarity agent" or "tonicity agent") generally refers to a substance that dissolves in the water of the composition of the invention and thus contributes to the osmolarity of the composition. Herein, the at least one osmolarity agent contributes the major part (i.e. more than 50 %) of the osmolarity (and osmolality) to the composition, e.g. at least 70 %, preferably at least 80 %, and more preferably at least 90 % of the osmolarity. The term "osmolarity agent" comprises both penetrating and non-penetrating solutes, i.e. solutes that can and that cannot penetrate a membrane of an osmotic cell. Examples of osmolarity agents are inorganic soluble salts such as sodium and potassium salts (e.g. sodium chloride, potassium chloride), mono- and disaccharides (e.g. trehalose), and sugar alcohols. Examples of sugar alcohols are glycerol, mannitol, xylitol, and sorbitol. One osmolarity agent may be used alone in the ophthalmic composition of the invention, or two or more may be used in combination. For example, inorganic soluble salts and sugar alcohols may be used in combination as the at least one osmolarity agent. Preferably, the at least one osmolarity agent is or comprises sodium chloride and optionally one or more sugar alcohol. In one embodiment, the at least one osmolarity agent is or comprises sodium chloride and glycerol.

The at least one osmolarity agent should be present in the ophthalmic composition of the invention so as to achieve the desired osmolarity. As indicated above, the ophthalmic composition of the present invention generally has an osmolarity of at least 700 mOsm/L or an osmolality of at least 700 mOsm/kg. The osmolarity is preferably at least 1000 mOsm/L, more preferably at least 1400 mOsm/L, even more preferably at least 1600 mOsm/L. The upper limit of the osmolarity is not specifically limited. However, it is generally not more than 2400, preferably not more than 2200 mOsm/L, more preferably not more than 2000 mOsm/L, to avoid excessive osmotic shock and/or burning sensation upon application to an eye. Accordingly, the osmolarity may be from 700 to 2200 mOsm/L, preferably from 1000 to 2000 mOsm/L, more preferably from 1400 to 2000 mOsm/L.

Alternatively, the osmotic pressure may be defined as osmolality. In such embodiments, the osmolality is preferably at least 1000 mOsm/kg, more preferably at least 1400 mOsm/kg, even more preferably at least 1600 mOsm/kg. The upper limit of the osmolarily is not specifically limited. However, it is generally not more than 2400, preferably not more than 2200 mOsm/kg, more preferably not more than 2000 mOsm/kg, to avoid excessive osmotic shock and/or burning sensation upon application to an eye. Accordingly, the osmolality may be from 700 to 2200 mOsm/kg, preferably from 1000 to 2000 mOsm/ kg, more preferably from 1400 to 2000 mOsm/kg.

If one of the at least one osmolarity agent is sodium chloride, the sodium chloride may be present in a concentration of from 2.0 to 6.5 % by weight, preferably from 3.0 to 6.0 % by weight, more preferably 4.0 to 5.5 % by weight, even more preferably from 4.5 to 5.3 % by weight, and most preferably about 5 % by weight. A content of the sodium chloride exceeding 6.0 % by weight is not preferred, since such content may cause excessive dehydration of ocular tissue such as the cornea. A content of the sodium chloride lower than 2.0 % by weight is not preferred, since such content may result in insufficient osmolarity for the desired dehydration effect. Within the ranges mentioned, eye irritation is suppressed to acceptable limits and good therapeutic effect can be achieved.

As mentioned above, the at least one osmolarity agent may be a combination of sodium chloride and a sugar alcohol, such as glycerol. The sugar alcohol may be present, in addition to the concentration of the sodium chloride, in a concentration of from 1.0 to 3.5 % by weight, preferably from 2.0 to 3.0 % by weight, and even more preferably from 2.3 to 2.7 % by weight of the ophthalmic composition. A content of the glycerol exceeding 3.5 % by weight is not preferred, since such content can cause excessive dehydration of ocular tissue, and a content of the glycerol below 1.0 % by weight (if present) is not preferred if such content results in insufficient osmolarity or osmolality for the dehydration effect. Within the ranges mentioned, sufficiently high osmolarity may be achieved while an excessive burning effect or eye irritation upon application of the ophthalmic composition to the eye can be avoided.

The hyperosmolar aqueous ophthalmic composition comprises carboxymethyl chitosan (herein sometimes abbreviated CMCh). Chitosan is a linear polysaccharide composed of randomly distributed β-(1→4)-linked D-glucosamine (deacetylated unit) and N-acetyl-D-glucosamine (acetylated unit). It is made by treating the chitin shells of shrimp and other crustaceans with an alkaline substance, such as sodium hydroxide. Another source of chitin is fungi. Chitosan is generally produced from chitin by full or partial deacetylation, whereby chitosans differ in the degree of deacetylation. Chitin is essentially insoluble in water. Deacetylation increases water solubility.

CMCh is produced from chitosan by carboxymethylation. Thus, CMChs also differ in the degree of deacetylation. There are, in principle, three positions per monomer unit for carboxymethylation: the (deacetylated) amino group in 2-position of glucose units and the hydroxy groups in 3 and 6 positions of the glucose units. Predominant carboxymethylation at the hydroxy groups is possible e.g. by protecting the amino groups. Since there may be remaining N-acetyl units from the original chitin and since free amino groups may be re-acetylated, there is a variability with regard to the content of N-acetyl units and carboxymethyl groups among CMChs. Formally, there is a maximum of three carboxymethyl groups per monomer unit. However, the "carboxymethylation reaction occurs preferably at C-6 hydroxyl groups and/or at the 2-amino groups, which allows producing N,O-carboxymethyl chitosan compounds that are water-soluble and comprise amino group as primary (-NH₂) or as secondary amine (-NH-CH₂COOH). (Zahra Shariatinia, International Journal of Biological Macromolecules, Volume 120, Part B, December 2018, pages 1406-1419).

The CMCh for use in the invention may have a degree of deacetylation of at last 30 %, preferably of at least 50 %, more preferably of at least 60 %, even more preferably at least 70 %, and most preferably of at least 80 % by mol. The deacetylation may be complete. However, since some N-acetyl groups generally remain, a preferred upper limit for deacetylation may be defined as 98 %. The percentage of deacetylation defines the mole percentage of glucosamine units having its 2-amino group not acetylated. Assuming complete deacetylation, the theoretical upper limit of carboxymethylation of the CMCh of the invention is 3 mol per monomer unit of said CMCh.

The degree of (remaining and/or re-introduced) acetylation (DA) or the degree of deacetylation may be measured by carbon-13 nuclear magnetic resonance (NMR) spectroscopy, as described in paragraph 0053 of US 2020/0390799 A1. Also, the degree of substitution (DS) by carboxymethyl groups (degree of carboxymethylation) may be measured according to the same disclosure.

The CMCh for use in the invention may have an average degree of carboxymethylation of at least 0.5, preferably at least 0.7, and more preferably at least 1.0 mol per monomer unit of said carboxymethyl chitosan. The CMCh may have an average degree of carboxymethylation from 0.5 to 2.5, preferably 0.7 to 2.0, more preferably 1.0 to 1.8 mol per monomer unit of said carboxymethyl chitosan. Average means the mole average over all CMCh present in a sample.

More specific embodiments are as follows in increasing level of preference:
the CMCh for use in the invention may have a degree of deacetylation of at last 60 % and an average degree of carboxymethylation from 0.5 to 2.5 mol per monomer unit of said CMCh;
a degree of deacetylation of at last 70 % and an average degree of carboxymethylation from 0.7 to 2.0 mol per monomer unit of said CMCh.

The ophthalmic composition generally comprises from 0.05 to 3 % by weight, preferably from 0.1 to 2 % by weight, more preferably from 0.1 to 1 % by weight, and most preferably from 0.1 to 0.5 % by weight of said CMCh. The weight of he CMCh is that of its theoretical neutral form, i.e. wherein carboxmethyl groups are assumed to be protonated (no negative charge) and any primary or secondary amino groups are deprotonated (no positive charge). Thus, counter-ions such as sodium or chloride are not counted as part of the weight of the CMCh.

The CMCh may have a molecular weight within the range of from 50 000 to 3 000 000 Dalton, preferable within the range of from 70 000 to 1 000 000 Dalton, more preferably within the range of from 90000 to 800 000 Dalton, and most preferably within the range of from 100 000 to 600 000 Dalton. The CMCh may have a weight average molecular weight of from 100,000 to 700,000, preferably from 150,000 to 500,000 Da. Generally, the molecular weight numbers relate to neutral CMCh, i.e. do not include the molecular weight of counter cations, such as sodium, in the case of carboxymethyl groups or chloride in the case of amino groups. The weight average molecular weight may be determined by size exclusion chromatography (SEC) in combination with a multi angle laser light scattering (MALLS) detector (SEC-MALS).

In preferred embodiments of increasing preference, the ophthalmic composition:
- comprises from 0.05 to 3 % by weight, preferably from 0.1 to 1 % by weight, and more preferably from 0.1 to 0.5 % by weight, of CMCh that
- may have molecular weight within the range of 90 000 to 800 000 Dalton or a weight average molecular weight of from 100,000 to 700,000,
- has a degree of deacetylation of at last 60 %, and
- has an average degree of carboxymethylation from 0.5 to 2.5 mol per monomer unit of said CMCh; or
- comprises from 0.05 to 3 % by weight, preferably from 0.1 to 1 % by weight, and more preferably from 0.1 to 0.5 % by weight, of CMCh that
- may have molecular weight within the range of 100 000 to 600 000 Dalton or a weight average molecular weight of from 150,000 to 500,000 Da,
- has a degree of deacetylation of at last 70 %, and
- has an average degree of carboxymethylation from 0.7 to 2.0 mol per monomer unit of said CMCh; or
- comprises from 0.1 to 1 % by weight, preferably from 0.1 to 0.5 % by weight, of CMCh that
- may have molecular weight within the range of 100 000 to 600 000 Dalton or a weight average molecular weight of from 150,000 to 500,000 Da,
- has a degree of deacetylation of at last 70 %, and
- has an average degree of carboxymethylation from 1.0 to 1.8 mol per monomer unit of said CMCh.

Where the ophthalmic composition is defined by a molecular weight range of the CMCh and a concentration thereof, any CMCh outside said molecular weight range does not contribute to the concentration of the CMCh.

The ophthalmic composition of the invention may further comprise one or more viscosity regulating agent. Herein, the viscosity regulating agent is other than the CMCh. The viscosity regulating agent of the invention may also be referred to as "thickener", "viscosity improver" or "gelling agent". The viscosity regulating agent is a polymeric hydrophilic compound that is soluble in water, thereby increasing the viscosity of the solution. It has film-building properties when applied to the eye, which protects the cornea and increases the remaining time of the ophthalmic composition on the cornea. The viscosity regulating agent may be a hydroxyalkylated cellulose, alkylated cellulose, chondroitin sulfate, polyacrylic acid, polyvinyl alcohol, polyethylene glycol, polysaccharides, polyvinylpyrrolidone, hyaluronic acid, cross-linked hyaluronic acid, or salts of any of these. The hydroxyalkylated cellulose may be hydroxypropylcellulose or hydroxyethylcellulose. The alkylated cellulose may be methylcellulose or ethylcellulose. Further, a viscosity regulating agent may be hydroxypropyl methylcellulose (hypromellose, HPMC), however, in one embodiment, the ophthalmic composition of the invention does not contain HPMC. Two or more viscosity regulating agents may be combined in the ophthalmic composition of the invention. Among the above viscosity regulating agents, hyaluronic acid or a salt thereof and cross-linked hyaluronic acid or a salt thereof are preferred and hyaluronic acid or a salt thereof is most preferred.

The ophthalmic composition of the invention may comprise the one or more viscosity regulating agent(s), preferably said hyaluronic acid and/or a hyaluronate, in a concentration of from 0.05 to 0.5 % by weight, preferably from 0.1 to 0.4% by weight, more preferably from 0.15 to 0.35 % by weight, and most preferably from 0.2 to 0.3% by weight of the ophthalmic composition. These concentration ranges relate to the sum of viscosity regulating agents (other than CMCh), if two or more different are used. Within the above range, the ophthalmic composition such as eye drop formulation is comfortable to apply and suppresses eye irritation. The exact concentration may be chosen so as to reach a desired viscosity of the ophthalmic composition. The viscosity regulating agent may be used alone or in combination of two or more kinds. Among the above, hyaluronic acid and salts thereof is particularly preferred from the view of film stability on the eye and suppression of eye irritation.

The molecular weight of the viscosity regulating agent may be chosen so as to adjust the viscosity of the ophthalmic composition to a desired range. The molecular weight may be within the range of from 100 000 to 10 000 000 Dalton, preferable within the range of from 500 000 to 5 000 000 Dalton, more preferably within the range of from 800 000 to 4 000 000 Dalton, and most preferably within the range of from 1 000 000 to 3 000 000 Dalton.

These molecular weights may be combined with the content of the one or more viscosity regulating agent(s) described above. In this embodiment, the content refers to the amount of the (one or more) viscosity regulating agent in the molecular weight range given. For example, the ophthalmic composition may contain from 0.05 to 0.5 % by weight of one or more viscosity regulating agent, preferably of hyaluronic acid and/or a hyaluronate, having a molecular weight within the range of from 100 000 to 10 000 000 Dalton, preferably within the range of from 500 000 to 5 000 000 Dalton, more preferably within the range of from 800 000 to 4 000 000 Dalton, and most preferably within the range of from 1 000 000 to 3 000 000 Dalton. In another example, the ophthalmic composition may contain from 0.2 to 0.4 % by weight of one or more viscosity regulating agent, preferably of hyaluronic acid and/or a hyaluronate, having a molecular weight within the range of from 100 000 to 10 000 000 Dalton, preferably within the range of from 500 000 to 5 000 000 Dalton, more preferably within the range of from 800 000 to 4 000 000 Dalton, and most preferably within the range of from 1 000 000 to 3 000 000 Dalton. Generally, the molecular weight numbers relate to hyaluronic acid, i.e. do not include the molecular weight of counter cations of hyaluronate, such as sodium.

The weight average molecular weight of the at least one viscosity regulating agent (preferably of the hyaluronic acid or hyaluronate) may be within the range of from 100 000 to 10 000 000 Dalton (100 kDa to 10 MDa), preferable within the range of from 500 000 to 5 000 000 Dalton (500 kDa to 5 MDa), more preferably within the range of from 800 000 to 4 000 000 Dalton (800 kDa to 4 MDa), and most preferably within the range of from 1 000 000 to 3 000 000 Dalton (1 MDa to 3 MDa). The weight average molecular weight may be determined by size exclusion chromatography (SEC) in combination with a multi angle laser light scattering (MALLS) detector (SEC-MALS).

In a preferred embodiment, the ophthalmic composition of the invention comprises hyaluronic acid or a salt thereof as the at least one viscosity regulating agent, and the weight average molecular weight of the hyaluronic acid or salt thereof is within the range of from 100 000 to 10 000 000 Dalton, preferable within the range of from 500 000 to 5 000 000 Dalton, more preferably within the range of from 800 000 to 4 000 000 Dalton, even more preferably within the range of from 1 000 000 to 3 000 000 Dalton, further more preferably within the range of from 1 300 000 to 2 600 000 Dalton, and most preferably within the range of from 1 500 000 to 2 000 000 Dalton. The weight average molecular weight may be determined by SEC-MALS.

As above, these weight average molecular weights may be combined with the content of the one or more viscosity regulating agent(s) described above. For example, the ophthalmic composition may contain from 0.1 to 0.5 % by weight of the one or more viscosity regulating agent (preferably said hyaluronic acid and/or a hyaluronate) and the weight average molecular weight of the at least one viscosity regulating agent is within any one of the ranges given in the previous paragraph. In another example, the ophthalmic composition may contain from 0.2 to 0.4 % by weight of the one or more viscosity regulating agent(s) (preferably said hyaluronic acid and/or a hyaluronate) and the weight average molecular weight of the at least one viscosity regulating agent is within any one of the ranges given in the previous paragraph.

GPC-MALLS is widely used to characterize high molecular weight (MW) polymers, which uses a differential refractometer and a multi-angle laser light scattering detector (MALLS) as detectors for gel permeation chromatogram (GPC). Separation of polymers is achieved by SEC based on different MW. The average molecular weight of an individual polymer is determined by MALLS based the differential scattering extent/angle of molecules of different MW. The GPC-MALLS method allows continuous measurement of the molecular weight and radius of gyration of each fraction separated by GPC. Principles of GPC-MALLS and protocols suited for hyaluronic acid are described by Ueno et al., 1988, Chem. Pharm. Bull. 36, 4971-4975; Wyatt, 1993, Anal. Chim. Acta 272: 1-40; and Wyatt Technologies, 1999, "Light Scattering University DAWN Course Manual" and "DAWN EOS Manual" Wyatt Technology Corporation, Santa Barbara, Calif.

The ophthalmic composition may further comprise heparin or a salt thereof. The heparin or a salt thereof may be contained in the ophthalmic composition in an amount of from 500 to 2000 I.U. / mL, preferably from 1000 to 1600 I.U. / mL, more preferably of from 1100 to 1500 I.U. / mL, even more preferably of from 1200 to 1400 I.U / mL, and even more preferably from 1250 to 1350 I.U. / mL of the ophthalmic composition. Heparin sodium is the preferred heparin salt.

Heparin is a sulfated glycosaminoglycan and thus a mucopolysaccharide. It is generally obtained from mucosal tissues of slaughtered animals, and is commercially available from various sources. The heparin for use in the invention may be unfractionated heparin, a low molecular weight heparin, an ultra-low molecular weight heparin, or a combination thereof. Examples of a low molecular weight heparin includes Bemiparin, Nadroparin, Reviparin, Enoxaparin, Parnaparin, Certoparin, Dalteparin, and Tinzparin. Preferably, the heparin for use in the present invention is selected from unfractionated heparin and low molecular weight heparin. Commercial sources for heparin sodium are, for example, Welding GmbH & Co KG, Midas Pharma GmbH, and Helm Portugal LDA.

As is common in the art, the amount or concentration of the heparin is defined in terms of the International units (I.U.) defined by the WHO based on its biological activity: one unit of heparin is the quantity of heparin required to keep one ml of cat's blood fluid for 24 hours at 0°C. The International Units are defined by the WHO. The relationship between the mass of the heparin and the international unit depends on the source and preparation methods of the heparin. "USP unit" means the United States Pharmacopeia Unit. The USP unit of heparin is harmonized with the international unit (IU) of heparin, effective October 1, 2009. Therefore, 1 USP unit heparin equals to 1 I.U. heparin. For example, one milligram of heparin obtained from Sigma-Aldrich (H3393) is at least 180 USP units or at least 180 I.U.

As described in WO 2022/063765 A1 (OmniVision GmbH), the presence of heparin or a salt thereof in a hyperosmolar ophthalmic composition can improve viability of corneal cells or tissue.

The ophthalmic composition may further contain a panthenol, such as dexpanthenol. The concentration of the panthenol in the ophthalmic composition may be from 0.2 to 3.0 % w/w dexpanthenol, preferably from 0.3 to 2.5 % w/w dexpanthenol. The presence of a panthenol may improve the wound healing properties of the ophthalmic composition.

The ophthalmic composition may, alternatively to the panthenol or additionally, comprise taurine. Taurine is 2-aminoethane-1-sulfonic acid. The skilled person understands that the protonation state and/or charge of taurine depends on the pH of the aqueous solution in which it is dissolved. Therefore, the term taurine comprises salts of taurine. However, where the concentration of taurine in the ophthalmic composition is given, the weight or weight percentage is in terms of the mass of 2-aminoethane-1-sulfonic acid. The concentration of taurine in the aqueous ophthalmic composition is, if present, generally from 0.1 to 3.0 % w/v, preferably from 0.2 to 2.5 % w/v taurine, more preferably from 0.3 to 2.0 % w/v of the aqueous ophthalmic composition.

The skilled person also understands that a panthenol and taurine contribute to the osmolarity (and osmolality) of the ophthalmic composition. Therefore, if the ophthalmic composition contains significant amounts of panthenol and/or taurine, the concentration of the at least one osmolarity agent may be reduced to achieve the same or similar osmolarity (and osmolality) as in the absence of the panthenol and/or taurine.

The ophthalmic composition of the present invention may further comprise an ophthalmically acceptable buffer. Examples of an ophthalmically acceptable buffer include citrate buffer, tris (trometamol) buffer, acetate buffer, histidine buffer, imidazole buffer, borate buffer, and phosphate buffer. Preferred buffers are citrate buffer and tris buffer, most preferred is tris buffer. A content of the buffer in the ophthalmic composition of the invention may be from 5 to 100 mM, preferably from 10 to 60 mM, more preferably from 15 to 50 mM, and even more preferably from 15 to 30 mM.

The ophthalmic composition of the present invention may have a pH value between 6.8 to 7.6, preferably between 7.0 and 7.4, more preferably between 7.1 and 7.3. In a preferred embodiment, the buffer of the ophthalmic composition is tris buffer in a concentration range of from 5 to 50 mM, preferably from 10 to 40 mM, and even more preferably from 10 to 30 mM and the ophthalmic composition has a pH within the range of from 7.1 to 7.3. In an exemplary embodiment, the buffer is 10 to 25 mM tris buffer pH at 7.2.

The ophthalmic composition is generally an aqueous ophthalmic composition. Thus, the ophthalmic composition contains water as solvent. Accordingly, the ophthalmic composition is generally liquid at room temperature (but may be semi-liquid in the case of a cream). Water as the solvent does not preclude the presence of other liquid components, such as glycerol indicated above.

The viscosity of the ophthalmic composition of the invention, notably when in the form of eye drops, may be, at a shear rate of 100 s⁻¹, from 2 to 1000 mPa s, preferably of from 3 to 500 mPa s, and more preferably from 4 to 200 mPa·s at 25°C, determined using a rotational viscometer. The skilled person understands that the viscosity may be set to a desired level by the content of CMCh and/or that of an optional viscosity regulating agent. Further, the viscosity is influenced by the molecular weight of said components.

The aqueous hyperosmolar ophthalmic composition of the invention may further contain suitable additives or pharmaceutical excipients. Examples of additives or excipients are stabilisers such as EDTA; non-ionic surfactants; or preservatives. Examples of preservatives frequently used in ophthalmic compositions are quaternary ammonium compounds such as benzalkonium chloride, benzethonium chloride, or polyquat. However, preferably, the ophthalmic composition of the invention does not contain a preservative, i.e. is preservative-free, more preferably it does not contain a quaternary ammonium compound as preservative. Any preservative present may be contained at less than 0.001 % by weight of a preservative, preferably of a quaternary ammonium compound preservative, such as benzalkonium chloride.

The ophthalmic composition of the invention may further contain an active pharmaceutical ingredient, such as an antibiotic, a vitamin, an antiphlogistic agent, an analgesic agent, and/or an agent for decreasing intra-ocular pressure or for treatment of glaucoma. Examples of an antibiotic are chloramphenicol, ofloxacin, norfloxacin, and neomycin. Examples of vitamins are retinol, retinol acetate, retinol palmitate, and tocopherol acetate. An example of an analgesic agent is ibuprofen. Examples of an agent for decreasing intra-ocular pressure or for treatment of glaucoma are bimatoprost, latanoprost, travoprost, brinzolamide, and timolol, or combinations thereof.

In a preferred embodiment, the hyperosmolar ophthalmic composition of the present invention comprises
(i) said carboxymethyl chitosan,
(ii) sodium chloride
(iii) optionally dexpanthenol
(iv) a buffer, and
(v) water,
wherein the osmolarity of the ophthalmic composition is at least from 1000 to 2000 mOsm/L or mOsm/kg. The CMCh may be as defined above, including the preferred embodiments described above (regarding degree of deacetylation and carboxymethylation, as well as molecular weight ranges).

In another preferred embodiment, the hyperosmolar ophthalmic composition of the present invention comprises
(i) said carboxymethyl chitosan,
(ii) sodium chloride
(iii) optionally dexpanthenol
(iv) heparin or a salt thereof
(v) a buffer, and
(vi) water,
wherein the osmolarity of the ophthalmic composition is at least from 1000 to 2000 mOsm/L or mOsm/kg. The CMCh may be as defined above, including the preferred embodiments described above (regarding degree of deacetylation and carboxymethylation, as well as molecular weight ranges).

In another embodiment, the ophthalmic composition of the present invention comprises
(i) from 0.05 to 3 % by weight, preferably from 0.1 to 1 % by weight, of said carboxymethyl chitosan,
(ii) from 3.0 to 6.5 % by weight, preferably from 3.5 to 6.0 % by weight sodium chloride,
(iii) optionally 0.2 to 3.0 % w/v dexpanthenol, preferably 0.3 to 2.5 % w/v dexpanthenol,
(iv) 5 to 100 mM, preferably 10 to 50 mM, of said buffer, and
(v) water.

In a further embodiment, the ophthalmic composition of the present invention comprises
(i) from 0.05 to 3 % by weight, preferably from 0.1 to 1 % by weight, of said carboxymethyl chitosan,
(ii) from 3.0 to 6.5 % by weight, preferably from 3.5 to 6.0 % by weight sodium chloride,
(iii) optionally 0.2 to 3.0 % w/v dexpanthenol, preferably 0.3 to 2.5 % w/v dexpanthenol,
(iv) heparin or a salt thereof in an amount of from 1000 to 1600 I.U. / mL,
(v) 5 to 100 mM, preferably 10 to 50 mM, of said buffer, and
(vi) water.

In both preceding embodiments, amounts or concentrations given relate to the total weight/weight of the ophthalmic composition. The CMCh may be as defined above, including the preferred embodiments described above (regarding degree of deacetylation and carboxymethylation, as well as molecular weight ranges). The osmolarity or osmolality may also as described above.

In an even more preferred embodiment, the ophthalmic composition of the present invention comprises
(i) from 0.05 to 3 % by weight, preferably from 0.1 to 1 % by weight, of said carboxymethyl chitosan,
(ii) from 4.0 to 5.5 % by weight, preferably from 4.5 to 5.3 % by weight sodium chloride,
(iii) 0.2 to 3.0 % w/v dexpanthenol, preferably 0.3 to 2.5 % w/v dexpanthenol,
(iv) 5 to 100 mM, preferably 10 to 50 mM, of said buffer, and
(v) water.

Amounts or concentrations given relate to the total weight/volume of the ophthalmic composition. The CMCh may be as defined above, including the preferred embodiments described above (regarding degree of deacetylation and carboxymethylation, as well as molecular weight ranges).

The aqueous hyperosmolar ophthalmic composition is for use in ophthalmology. The ophthalmic composition may be for use in a method of treating or reducing corneal swelling or fluid accumulation of any origin, including stromal corneal edema or epithelial corneal edema, or restoring osmotic balance of the cornea, especially after surgical intervention. In one embodiment, the ophthalmic composition is for use in the treatment of corneal edema. The corneal edema may be stromal corneal edema, epithelial corneal edema, corneal osmotic imbalance, post-surgical corneal osmotic imbalance, and corneal swelling or fluid accumulation of any origin. The ophthalmic composition is preferably for use in a method of treating corneal edema and of preventing or reducing the risk of bacterial infection of the cornea. The ophthalmic composition is, alternatively, for use in a method of treating corneal edema and of preventing or reducing the risk of bacterial growth on the cornea.

Furthermore, the present invention provides a method of treating corneal edema, including stromal corneal edema, epithelial corneal edema, corneal osmotic imbalance, especially post-surgical corneal osmotic imbalance, and corneal swelling or fluid accumulation of any origin, the method comprising using the ophthalmic composition of the invention, preferably administering the ophthalmic composition to an affected eye of a patient. The invention preferably provides a method of treating corneal edema and of preventing or suppressing bacterial infection (or decreasing the risk of bacterial infection) of the cornea, and/or a method of preventing or suppressing bacterial growth on the cornea, the method comprising using the ophthalmic composition of the invention, preferably administering the ophthalmic composition to an affected eye of a patient.

Corneal edema is a common sign of acute or protracted corneal disease. The causes of corneal edema leading to the loss of physical barrier, loss of pump function or increased intraocular pressure, can be classified into four groups: mechanical, toxic, dystrophic and inflammatory. The mechanical cause can be further divided into traumatic and glaucomatous. The traumatic cause could be incidental or surgical. The trauma can be categorized into non-penetrating trauma and penetrating trauma. For example, ocular surgery, which is a penetrating surgical trauma, often leads to corneal edema.

The ophthalmic composition of the present invention is generally applied topically to a subject's eye affected by any of the indications mentioned above. The ophthalmic composition may be applied to an eye, such as the conjunctival sac of an eye, of a subject in need thereof. The composition may be applied in a single dose. However, preferably, it is applied two or more times per day, preferably three to five times per day. It may be applied to an eye one or more times per day over a period of from one day to several weeks (e.g. to four weeks), such as from one day to two weeks or from two days to seven days. For certain indications such as for corneal dystrophy (e.g. Fuchs' dystrophy), the ophthalmic composition may be applied for periods even longer than four weeks, e.g. if treatment by surgery is not possible or desired. In an embodiment, the ophthalmic composition is applied three to five times per day over a period of from 2 to 14 days. If the ophthalmic composition is formulated as an eye drop formulation or ophthalmic gel, it may be applied by dropwise instillation into an eye, such as into the conjunctival sac of an eye, affected by any one of the above indications. In the form of a cream or ointment, the ophthalmic composition may be applied to the surface of an eye, such as into the conjunctival sac of an eye.

The ophthalmic composition of the invention may be formulated, for example, as eye drops, as an ophthalmic gel, or ophthalmic cream. Preferably, the ophthalmic composition is formulated as eye drops or gel and is applied dropwise to an eye of a subject.

The ophthalmic composition of the invention may be produced through a customary process by dissolving the osmolarity agent and the CMCh and optionally any desired further component (such as an additional viscosity regulating agent, taurine and/or a buffer) in water, adjusting the pH with, for example hydrochloric acid or sodium hydroxide, and adding water up to the desired volume to achieve the aqueous composition with the components in the desired concentrations. Preferably, separate solutions in water of CMCh and an additional viscosity regulating agent, and optionally excipients such as buffer, may be prepared and added to a solution of an osmolarity agent and any further components; then adjusting the pH with, for example hydrochloric acid or sodium hydroxide, and adding water up to the desired volume. The resultant solution may then be sterilized, e.g. by sterile filtration, before being packed in suitable plastic containers.

A hyperosmolar ophthalmic gel as the ophthalmic composition of the invention may be produced as follows. Water for injection is heated, sodium chloride as an osmolarity agent and optionally taurine is added, optionally other components and a buffer is added. The solution is stirred and filtered through a microporous membrane. After heating up again, any additional osmotic regulator, CMCh and an optional additional viscosity regulating agent as gel matrix are added while hot, the mixture is stirred to reach room temperature. Then water to the desired final volume (e.g. 1000 ml) is added and the mixture is homogenized. A terminal sterilization may be conducted to obtain a uniform and sterile gel.

A hyperosmolar ophthalmic ointment as the ophthalmic composition of the invention may be produced as follows. A hot aqueous phase containing sodium chloride as an osmolarity agent and CMCh and, if used, an additional viscosity regulating agent component is prepared. A hot phase containing fats and an emulsifying agent is prepared separately. The aqueous phase is added to the fat phase and the mixture is homogenized and cooled. A terminal sterilization may be conducted to obtain a uniform and sterile product.

### EXAMPLES

The present invention will next be described in more detail by way of Examples, which should not be construed as limiting the invention thereto.

### Measurement of dynamic viscosity

The determination of the dynamic viscosity for the ophthalmic solutions is carried out by means of a rotational concentric cylinder viscometer (Searle type) at 20°C.

### Materials

- Metallic cylinder supplied with the viscosimeter
- Rotational viscosimeter Visco
- Hook for the UL spindle
- adequate UL spindle

### Method

- Temperature 20°C.
- Pour into the 16ml metallic cylinder the test solution.
- Insert the cylinder in the dedicated section on the base of the viscosimeter.
- Connect the UL spindle to the hook and fix the complex to the stand of the viscosimeter.
- Connect the temperature sensor to the viscosimeter.
- Place the viscosimeter onto the base.
- Turn on the instrument pushing the multi-functional button on the back side.
- In the main menu select the item LEVEL to enter in the instrument level mode to ensure the correct adjustment of the level and if not proper, set the correct level rotating the appropriate stand screws on the base of the instrument.

- Enter in the section MEASURMENT to set the UL spindle.
- Start the measurement from the lower speed (expressed in rpm) set between 100 and 250 rpm. The instrument emits an acoustic signal in case of error, so increase the speed more and more in reference to the displayed value; the analysis result will be the one with the torque value nearest to the 100%.
The measurement is performed three times with the same sample. Reported values are the average of the three measurements.

### Measurement of osmolality

Osmolality is determined by measuring freezing point depression according to Eur. Pharm. 2.2.35. A micro-osmometer Löser Type6/6M was used. The method is carried out as described by the manufacturer as follows:
1. Turn on the instrument and wait for the acoustic signal.
2. Fill the centrifuge tube (1.5 mL) with 100 µl of test solution and place it in the thermistor (temperature dependent resistor).
3. Pull down the thermistor in the cooling hole.
4. The value of the decreasing temperature is displayed. It ensures the progressive freezing. After 1.5 minutes the acoustic signal indicates that the freezing point is reached.
5. Raise completely the freezing needle and insert it into the tube. After 1 second replace the needle to original position.
6. Wait for the acoustic signal and record the value shown on the display.
7. Remove the sample tube.
The measurement is performed three times with the same sample. Reported values are the average of the three measurements.

### COMPARATIVE EXAMPLE 1: Production of ophthalmic compositions comprising chitosan or CMCh

The impact of sodium chloride, namely 0.0 %, 1.0 %, 2.5 % and 5.0 % on different chitosan derivates (Chitosan-HCl as well as CMCh in the concentration 0.2 % and 0.5 %) was investigated. For this purpose, various formulations as listed in Tab. 1 were prepared and their appearance was observed over time. Differences in the appearance could be observed after one week of storage. While CMCh resulted in clear solution even in the presence of 5.0% sodium chloride, turbidity was formed with chitosan-HCl in the presence of only 0.5 % NaCl. The outcome indicates that Chitosan-HCl is more sensitive compared to CMCh underlying by no turbidity over time independent of the salt concentration.

| **Appearance after preparation** | **0.0% NaCl** | **0.5% NaCl** | **1.0% NaCl** | **2.5% NaCl** | **5.0% NaCl** |
|---|---|---|---|---|---|
| Chitosan-HCl 0.2% | Clear solution, no turbidity | Clear solution, no turbidity | Clear solution, no turbidity | Clear solution, no turbidity | Clear solution, no turbidity |
| Chitosan-HCl 0.5% | Clear solution, no turbidity | Clear solution, no turbidity | Clear solution, no turbidity | Clear solution, no turbidity | Clear solution, no turbidity |
| CMCh 0.2% | Clear solution, no turbidity | Clear solution, no turbidity | Clear solution, no turbidity | Clear solution, no turbidity | Clear solution, no turbidity |
| CMCh 0.5% | Clear solution, no turbidity | Clear solution, no turbidity | Clear solution, no turbidity | Clear solution, no turbidity | Clear solution, no turbidity |

| **After one week storage** | | | | | |
|---|---|---|---|---|---|
| Chitosan-HCl 0.2% | Slight turbidtiy of the solution | Slight turbidtiy of the solution | Turbidity of the solution | Turbidity of the solution | Turbidity of the solution |
| Chitosan-HCl 0.5% | Slight turbidtiy of the solution | Turbidity of the solution | Turbidity of the solution | Turbidity of the solution | Turbidity of the solution |
| CMCh 0.2% | Clear solution, no turbidity | Clear solution, no turbidity | Clear solution, no turbidity | Clear solution, no turbidity | Clear solution, no turbidity |
| CMCh 0.5% | Clear solution, no turbidity | Clear solution, no turbidity | Clear solution, no turbidity | Clear solution, no turbidity | Clear solution, no turbidity |

| **After two weeks storage** | | | | | |
|---|---|---|---|---|---|
| Chitosan-HCl 0.2% | Phase seperation; white streaks and clear supernatant | Phase seperation; white streaks and clear supernatant | Phase seperation; white streaks and clear supernatant | Phase seperation; white streaks and clear supernatant | Phase seperation; white streaks and clear supernatant |
| Chitosan-HCl 0.5% | Phase seperation; white streaks and clear supernatant | Phase seperation; white streaks and clear supernatant | Phase seperation; white streaks and clear supernatant | Phase seperation; white streaks and clear supernatant | Phase seperation; white streaks and clear supernatant |
| CMCh 0.2% | Clear solution, no turbidity | Clear solution, no turbidity | Clear solution, no turbidity | Clear solution, no turbidity | Clear solution, no turbidity |
| CMCh 0.5% | Clear solution, no turbidity | Clear solution, no turbidity | Clear solution, no turbidity | Clear solution, no turbidity | Clear solution, no turbidity |

### REFERENCE EXAMPLE 1: Production of an ophthalmic composition comprising CMCh

The formulation listed below is prepared according to following main process steps:
- Add to a suitable vessel step-by-step (waiting for full dissolution before adding the next component) 25 mM Tris buffer, 5% NaCl and 2% dexpanthenol.
- Add 0.2% CMCh to the other components and stir until complete dissolution
- Measurement of the pH value
- If necessary: Adjustment of the pH Vale to the range of 6.8-7.2 utilizing NaOH and HCl
- Stirring of the solution until complete dissolution

| **Ingredients** | **Quantity** |
|---|---|
| Carboxymethyl chitosan | 0.2 % w/w |
| Sodium Chloride | 5.0 % w/w |
| Dexpanthenol | 2.0 % w/w |
| TRIS | 25 mM |
| NaOH/HCl | q.s. pH 7.2 |
| Water for Injections | q.s. 100 % |

The CMCh used was N,O-carboxymethyl chitosan (CAS: 83512-85-0) obtained from Heppe Medical Chitosan GmbH, Halle (Saale), Product-No: 44002, the deacetylation degree was 94 %, molecular weight range (by GPC): 30-500 kDa; viscosity (1 % solution at 20 °C): 31 mPa·s.

### EXAMPLE 1: Antimicrobial efficacy of different eye drop formulations against the bacterial test strains Staphylococcus aureus ATCC 6538 and Pseudomonas aeruginosa ATCC 9027

In order to investigate the antimicrobial efficacy of CMCh formulations, turbidimetric determination of a bacterial growth curve in different formulations and corresponding diluents were preformed utilizing *Pseudomonas aeruginosa* ATCC 9027 and *Staphylococcus aureus* ATCC 6538.

The test strain *S*. *aureus* ATCC 6538 is incubated in CaSo-M (soya bean casein digest Medium, Merck 1.05459) at 30-35 °C for about 24 h in sterile screw-capped glass tube without shaking (preculture). For preparation of inoculum suspensions, an appropriate volume of the *S*. *aureus* preculture was added to the required volume of CaSo-M (e.g. 35 mL) to obtain an optical density of about 0.1 (at wavelength 540 nm).

The test strain *P*. *aeruginosa* ATCC 9027 is incubated in CaSo-M at 30-35 °C for about 24 h in a sterile glass flask under shaking (preculture). For preparation of an inoculum suspension, an appropriate volume of the *P*. *aeruginosa* preculture will be added to the required volume of CaSo-M (e.g. 70 mL) to obtain an optical density of about 0.1 (at 540 nm).

The following test solutions were prepared:
- 0.9 % by weight NaCl in water,
- 5 % by weight NaCl in water,
- 1 % by weight CMCh in 0.9 % by weight aqueous NaCl, pH 7.2
- 0.2 % by weight CMCh in 5 % by weight aqueous NaCl, pH 7.2

The CMCh used is as described in Reference Example 1.

With *P*. *aeruginosa,* the test was performed in sterile glass flask under shaking by using an orbital shaker at 150-200 rpm. 10 mL of each test solution was transferred to a sterile 100 mL glass flask using a sterile pipette. Then, 10 mL of inoculum suspension in CaSo-M was added to each glass flask containing the test solution, resulting in a final OD₅₄₀ₙₘ of about 0.05 and a 2-fold dilution of test solution and CaSo-M. In order to obtain negative controls/blank, 10 mL of each test solution was transferred to a sterile glass flask, followed by adding 10 mL of CaSo-M without test strain. Initial optical densities of the inoculated test solution-medium mixtures were measured using a cuvette with a spectrophotometer at 540 nm. The corresponding test solution-medium mixtures without test strain served as blank. The inoculated test solution-medium mixtures and the negative controls/blank were incubated at 30-35 °C without shaking. Optical density was measured every 60 min (or less) for a duration of 6-7 h using a cuvette. The final measurement was taken after 24 h of incubation and afterwards the experiment was stopped. At OD values > 0.5, the suspension were diluted accordingly before OD measurement.

In case of *S*. *aureus,* the test was performed according to the same protocol, but in sterile screw-capped glass tubes without shaking.

### Results

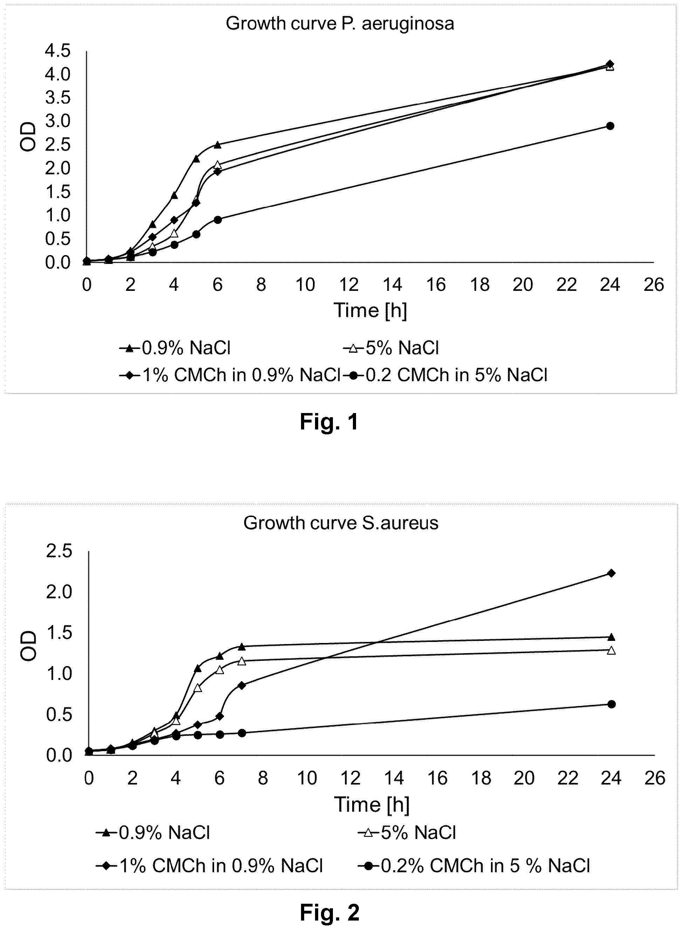

As shown in Fig. 1, the 5 % NaCl test solution moderately inhibited growth of *P*. *aeruginosa* ATCC 9027 compared to the 0.9 % NaCl test solution. Also, the test solution containing 1 % CMCh and 0.9 % NaCl moderately inhibited bacterial growth, roughly to a similar extent as the 5 % NaCl solution.

However, the test solution containing 0.2% CMCh and 5% NaCl resulted in a much stronger growth inhibition of *P*. *aeruginosa* ATCC 9027 than the 5% NaCl solution, that was even higher than the sum of the individual growth inhibitions of the 5 % NaCl solution and the 1 % CMCh, 0.9 % NaCl solution. This strong effect with the 0.2% CMCh, 5% NaCl test solution was achieved although the CMCh concentration was lower than in the 1 % CMCh, 0.9 % NaCl test solution.

The trends observed with *P*. *aeruginosa* ATCC 9027 were confirmed with *S*. *aureus* ATCC 6538 (Fig. 2).

Thus, a hyperosmolar CMCh-containing ophthalmic solution is highly advantageous for use as eye drops in order to suppress bacterial growth in or on the eye, e.g. after ocular surgery, and can at the same time treat ocular edema due being hyperosmolar.

### Formulation Examples

### Formulation Example 1

| **Ingredient** | **Quantity** |
|---|---|
| Carboxymethyl chitosan | 0.5% w/w |
| Sodium Chloride | 5.0% w/w |
| Dexpanthenol | 2.0% w/w |
| TRIS | 25 mM |
| NaOH/HCl | q.s. pH 7.2 |
| Purified Water or Water for Injections | q.s. 100 % |

### Formulation Example 2

| **Ingredient** | **Quantity** |
|---|---|
| Carboxymethyl chitosan | 0.75% w/w |
| Sodium Chloride | 5.0% w/w |
| Dexpanthenol | 2.0% w/w |
| TRIS | 25 mM |
| NaOH/HCl | q.s. pH 7.2 |
| Purified Water or Water for Injections | q.s. 100 % |

### Formulation Example 3

| **Ingredient** | **Quantity** |
|---|---|
| Carboxymethyl chitosan | 0.2% w/w |
| Sodium Chloride | 4.5 % w/w |
| Dexpanthenol | 2.0% w/w |
| TRIS | 25 mM |
| NaOH/HCl | q.s. pH 7.2 |
| Water for Injection | q.s. 100 % |

### Formulation Example 4

| **Ingredient** | **Quantity** |
|---|---|
| Carboxymethyl chitosan | 0.5% w/w |
| Sodium Chloride | 4.5 % w/w |
| Glycerol | 1.0% w/w |
| TRIS | 25 mM |
| NaOH/HCl | q.s. pH 7.2 |
| Water for Injection | q.s. 100 % |

### Formulation Example 5

| **Ingredient** | **Quantity** |
|---|---|
| Carboxymethyl chitosan | 1.0% w/w |
| Sodium Chloride | 4.5 % w/w |
| Taurine | 1.0% w/w |
| TRIS | 25 mM |
| NaOH/HCl | q.s. pH 7.2 |
| Water for Injection | q.s. 100 % |

### Formulation Example 6

| **Ingredient** | **Quantity** |
|---|---|
| Carboxymethyl chitosan | 1.0% w/w |
| Sodium Chloride | 4.0 % w/w |
| Dexpanthenol | 2.0% w/w |
| Taurine | 1.0% w/w |
| TRIS | 25 mM |
| NaOH/HCl | q.s. pH 7.2 |
| Water for Injection | q.s. 100 % |

### Formulation Example 7

| **Ingredient** | **Quantity** | |
|---|---|---|
| Carboxymethyl chitosan | 0.5% w/w | |
| Sodium Chloride | 4.5 % w/w | |
| Glycerol | 1.0% w/w | |
| Heparin sodium, Ph. Eur. | 6.5 mg/mL (≙1300 I. U.) | |
| TRIS | 25 mM | |
| NaOH/HCl | q.s. pH 7.2 | |
| Water for Injection | q.s. 100 % | |

The same CMCh as described in Reference Example 1 is used in these Formulation Examples.

## Claims

1. A hyperosmolar ophthalmic composition comprising carboxymethyl chitosan.

2. The hyperosmolar ophthalmic composition according to claim 1, comprising from 0.05 to 3 % by weight, preferably from 0.1 to 1 % by weight, of said carboxymethyl chitosan.

3. The ophthalmic composition according to claim 1 or 2, wherein said carboxymethyl chitosan has a degree of deacetylation of at least 50 %, preferably from 60 to 98 % by weight.

4. The ophthalmic composition according to any one of claims 1 to 3, wherein said carboxymethyl chitosan has an average degree of carboxymethylation of from 0.5 to 2.5, preferably 0.7 to 2.0, more preferably 1.0 to 1.8 mol per monomer unit of said carboxymethyl chitosan.

5. The ophthalmic composition according to any one of claims 1 to 4, further comprising a buffer selected from citric acid, tris, histidine, imidazole, and phosphoric acid, and/or salts thereof, preferably tris or a salt thereof.

6. The ophthalmic composition according to any one of claims 1 to 5, further comprising a panthenol, such as dexpanthenol.

7. The ophthalmic composition according to any one of claims 1 to 6, further comprising at least one viscosity regulating agent that may be selected from the group consisting of a hydroxyalkylated cellulose, chondroitin sulfate, polyacrylic acid, polyvinyl alcohol, polyethylene glycol, polysaccharides, polyvinylpyrrolidone, heparin, hyaluronic acid, cross-linked hyaluronic acid, and salts thereof, preferably wherein the one or more viscosity regulating agent is or comprises hyaluronic acid or a salt thereof.

8. The ophthalmic composition according to any one of claims 1 to 7, comprising at least one osmolarity agent.

9. The ophthalmic composition according to claim 8, wherein the at least one osmolarity agent is selected from the group consisting of sodium chloride, potassium chloride, trehalose, and sugar alcohols.

10. The ophthalmic composition according to any one of claims 1 to 9, wherein the ophthalmic composition is an aqueous solution having a osmolarity of at least 1000 mOsm/L, preferably at least 1400 mOsm/L.

11. The ophthalmic composition according to any one of claims 1 to 10, comprising:
(i) said carboxymethyl chitosan,
(ii) sodium chloride
(iii) dexpanthenol,
(iv) a buffer,
(v) water,
wherein the osmolarity of the ophthalmic composition is at least 1000 mOsm/L.

12. The ophthalmic composition according to any one of claims 1 to 11, having a pH of from 6.5 to 7.6; and/or a dynamic viscosity of, at a shear rate of 100 s⁻¹, from 2 to 1000 mPa s, preferably of from 3 to 500 mPa s, and more preferably from 4 to 200 mPa·s at 25°C, determined using a rotational viscometer.

13. The ophthalmic composition according to any one of claims 1 to 12, which contains no or less than 0.001 % by weight of a preservative such as benzalkonium chloride.

14. The ophthalmic composition according any one of the preceding claims for use in ophthalmology, preferably for use in a method of treating or reducing corneal swelling or fluid accumulation of any origin, including cataract surgery, corneal transplantation, stromal corneal edema or epithelial corneal edema, or for restoring osmotic balance of the cornea, especially after surgical intervention such as cataract surgery or corneal transplantation.

15. The ophthalmic composition according any one of the preceding claims for use in a method of treating corneal edema and of preventing or reducing the risk for bacterial infection and/or of bacterial growth on the cornea.
